# EUROPEAN PATENT SPECIFICATION

(11) **EP 1 734 938 B1**
(45) Date of publication and mention of the grant of the patent: **20.06.2012**
(21) Application number: 04723612.0
(22) Date of filing: 26.03.2004
(51) Int. Cl.: A61K 9/72, A61K 9/16, A61K 38/28

(54) **INSULIN HIGHLY RESPIRABLE MICROPARTICLES**
STARK EINATEMBARE INSULIN-MIKROTEILCHEN
MICRO-PARTICULES A BASE D'INSULINE ET HAUTEMENT INHALABLES

(43) Date of publication of application: 27.12.2006
(73) Proprietor: UNIVERSITA' DEGLI STUDI DI PARMA, 43100 Parma (IT)
(72) Inventor: COLOMBO, Paolo, I-43100 Parma (IT); CAGNANI, Stefano, I-29020 Settima di Gossolengo (IT); VENTURA, Paolo, I-29100 Piacenza (IT)
(74) Representative: Gervasi, Gemma
(86) International application number: PCT/EP2004/050371
(87) International publication number: WO 2005/092301

(56) References cited:
- EP-A- 0 505 966
- WO-A-01/93837
- WO-A-99/55362
- WO-A-02/053190
- FORBES R T ET AL: "WATER VAPOR SORPTION STUDIES ON THE PHYSICAL STABILITY OF A SERIES OF SPRAY-DRIED PROTEIN/SUGAR POWDERS FOR INHALATION" JOURNAL OF PHARMACEUTICAL SCIENCES, AMERICAN PHARMACEUTICAL ASSOCIATION. WASHINGTON, US, vol. 87, no. 11, November 1998 (1998-11), pages 1316-1321, XP000783384 ISSN: 0022-3549
- TODO HIROAKI ET AL: "Effect of additives on insulin absorption from intratracheally administered dry powders in rats" INTERNATIONAL JOURNAL OF PHARMACEUTICS (KIDLINGTON), vol. 220, no. 1-2, 4 June 2001 (2001-06-04), pages 101-110, XP002310507 ISSN: 0378-5173
- QUAGLIA F ET AL: "Feeding liquid, non-ionic surfactant and cyclodextrin affect the properties of insulin-loaded poly(lactide-co-glycolide) microspheres prepared by spray-drying" JOURNAL OF CONTROLLED RELEASE, ELSEVIER SCIENCE PUBLISHERS B.V. AMSTERDAM, NL, vol. 86, no. 2-3, 17 January 2003 (2003-01-17), pages 267-278, XP004401121 ISSN: 0168-3659

## Description

### FIELD OF THE INVENTION

It is known that certain drugs delivered to the lung are readily absorbed through the alveolar region into the blood circulation. Pulmonary delivery is a promising alternative route in particular for the administration of macromolecules such as proteins, polypeptides and nucleic acids, which are exclusively delivered by injection. Lung delivery is useful for both systemic and local therapeutic activity. Pulmonary drug delivery has to be achieved by producing an aerosol of the active. Aerosols can be generated by different methods, including liquid nebulizers, pressurized metered dose inhalers (MDI) and dry powders inhalers (DPI). The CFC propellant phase out caused aerosol based MDI to loose favor while increasing interest focused on dry powder devices. In such devices, drugs are formulated as respirable dry powder obtained by freeze-drying or spray-drying or other suitable techniques. The drugs may be combined with safe excipient in order to improve their respirability, stability and flowability.

Typical techniques for delivering dry powder formulations via a DPI are loading one dose of the drug in a hard gelatin capsule or aluminum blister or to load the device with multiple doses leaving to the device to sample the amount required. This step requires powders having favorable properties of flow and packing. These properties are typical of the coarse powders. Upon patient inspiration the air flowing through the device penetrates into the drug reservoir and aerosolizes the powder dose. This second step requires micronized powders having particle with size, shape and density useful for aerosolization.

The ability to deliver a drug to the alveolar region of the respiratory tract, where the absorption takes place, is problematic under different points of view. In details, a powder must be capable to overcome the paradox to be fine for aerosolization and lung deposition but at the same time to be coarse for the dosing in the device of the amount to be administered. Firstly, the dose of drug must be accurately metered and this relies on the packing and flow properties of the powder and characteristics of device for administration. Secondly, the powder to be inhaled must be easily dispersed in order to assure the generation of respirable aerosol and this relies on powder size, shape and density. A solution for these two contradictory aspects is the object of this patent.

### DESCRIPTION OF RELEVANT LITERATURE

The delivery of insulin to the lung has been proposed since its discovery. The simplest method to deliver insulin to the lung in preclinical studies was by direct intra-tracheal instillation of an aqueous formulation. In this case, distribution in the lung tends to be focalized and uneven than that seen after aerosol administration, resulting in small surface area available for the absorption. A. L. Jones (Proceedings of the third European congress of Biopharmaceutics and Pharmacokinetics, Vol 2, page 143-149) in 1987 reported a rapid absorption of insulin in rats. P. Colthorpe and S. Farr in 1992 (Pharmaceutical Research, 9: 764-768) using pharmaco-scintigraphy compared the deposition and absorption of the direct instillation and the nebulization of an acidic insulin solution. He elegantly demonstrated that the absorbed fraction for aerosolized insulin was 10 fold greater than instilled insulin. This provides a dear proof how the mode of administration profoundly affects the fate of pulmonary delivery.

Using intratracheal instillation, the effect of formulation related variables on the extent of pulmonary insulin absorption has also been investigated, including osmolarity, viscosity and solution pH. According to F. Komoda (J. Pharm. Sci. 1994, 83, 863-867) insulin formulation at pH 3 showed greater bioavalability than those at pH 7 after intratracheal instillation. They explained the result by the inhibition of insulin association in the lower pH formulation. Insulin exists as monomer, dimer and exhamer. Dimers and exhamers come from aggregation of the monomeric form and the relative percentage in solution of three forms depends on pH and concentration of the hormone. It is known that insulin self-aggregation affects its diffusive transport across biological membrane (Diabetes Care 1990, 13, 953-954).

Many ways have been suggested for aerosolizing insulin in form of solution, dry powders and even suspension of liposomes. Metered dose inhalers and Dry powder inhalers are the most recent devices for pulmonary administration of drugs. Metered dose inhalers for delivering crystalline insulin suspended in a propellant have been proposed by S. Lee (J. Pharm. Sci. 1976: 65, 567-572) and a patent exists on this field (US 5320094). Dry powders inhalers carrying insulin are also described in the literature (for a complete review: J.S. Patton: Inhaled Insulin, Adv. Drug Del. Rev. 35, 1999, 235-247). Pulmonary delivery of dry powder medicaments in large particle porous particles has been investigated by R. Langer and co-workers (J. App. Physiol. 1998: 85, 379-385), and patented (WO 9966903). Others preparations for inhalation which comprise insulin and a substance which enhances the absorption of insulin in the lower respiratory tract, have been proposed in the form of a powder preparations suitable for inhalation (US6306440). Intranasal and respiratory delivery of a variety of polypeptides, including insulin, in the presence of an enhancer has been also described by T. Nagai (J. Contr. Rel. 1984: 1, 15-22) and L. Rydén (Int. J. Pharm. 1982: 83, 1-10) and in several patents released worldwide (WO 9302712, WO 9102545, WO 9009780, WO 8804556).

The preparation of an amorphous powder containing insulin was illustrated in a patent which described the methods for spray drying polypeptide, polynucleotide and other liable drugs together with a carrier to improve stability of the active after drying (EP 0520748). The first patent on insulin medical aerosol formulation (EP 0655237) discloses the production of an aerosol containing also spray dried insulin intended for lung administration. In the example 4 of the cited patent a powder is prepared from an alcoholic (25% v/v) solution containing the same amount of insulin and lactose and 0.1% soya bean lecithin. In another patent (WO A 9524183) insulin is produced in form of a dried powder from buffered solution at pH 6.7 ± 0.3 containing the hormone. This patent discloses the use of a citrate buffer to dissolve crystal insulin and subsequently the powder is produced by spray drying. Some criticism (refers to WO 00/00176, page 2, line 4 - line 6) has been addressed to the experimental section.

Insulin powders for inhalation, prepared by spray drying a solution of insulin in an acidic buffer, are described by RT Forbes (J. Pharm. Sci. 1998: 87, 1316-1321), by H Todo (Int. J. Pharm. 2001: 220, 101-110) and in patent applications (WO 02053190, WO 0193837).

Finally, dry powders inhalers are disclosed in several patent applications. Manual pumps (US 3921637) or multiple receptacle disks or strips (EP 0467172) are employed. Puncturing gelatin capsule disperser is described somewhere else (US 43389314). A held-pump device has been also patented (WO 09007351). Independently on the device used, the characteristics of drug in powder form are crucial for the efficacy of the preparation.

### OBJECT OF THE INVENTION

The object of present invention are pharmaceutical powders of insulin suitable for pulmonary administration intended for the long-term treatment of diabetes, characterized by a structure of the microparticles composing the powder that imparts an elevated respirability, together with favorable flow and packing characteristics. The powders obtained through have been produced by spray drying in never explored conditions of manufacturing, in particular at pH lower than the isoelectric point of insulin. The acidic pH used for particle preparation would allow to obtain higher absorption as F. Komoda previoulsy demonstrated.

The invention relates to microparticles stable at room temperature of insulin, optionally in association with excipients selected from the group consisting of saccharides, polysaccharides, aminoacids, phospolipids and polyalcohol, said microparticles:
- being obtained by spray drying a clear aqueous solution of insulin having an acid pH under the isoelectric point (5.4) of insulin and a concentration of insulin in amounts of from 5 to 100 mg/ml,
- showing a d90 volume diameter lower than 9 µm,
- more than 80% of them exhibiting an aerodynamic diameter lower than 5 µm,
- containing less than 10% by weight of salts,
characterised in that said aqueous solution of insulin to be spray dried is prepared in an unbuffered aqueous solution of acetic acid.

The pulmonary powders of insulin object of this patent are characterized by structure and shape of the constituent microparticles defined corrugated or raisin like, completely different from the insulin microcrystal structure. These pulmonary powders of insulin show a flow and packing characteristics that allow them to be directly introduced in the reservoir of DPI delivery systems. More relevant is the fact that the respirability of the powders having these properties is higher that the usual values described in literature. In fact, the fraction of respirable particles composing the insulin aerosol produced with the powders here described ranged between 83.9% and 90.4%, whereas common values range between 20-40 %. This respirability has been assessed using the Andersen Cascade Impactor as described in the European Pharmacopeia (4th Edition, <2.9.18> page 216). This apparatus is used to determine the fine particles of an aerosol cloud, generated by preparations for inhalation, and allows the measure of the mass of drug less than a particular aerodynamic particle size. The mass of drug having aerodynamic diameter lower than 5 µm is generally considered as "respirable", even though the optimal size for alveolar deposition is in the range 5 - 2 micron.

The manufacturing procedures described in this patent provide the production of fine powders in which more than 90 % of particles have dimensions less than 9 microns as volume diameter. The method for preparing said formulation allows high percentage of pharmaceutical activity of the powder to be manufactured. Moreover, the dry powder exhibits adequate chemical and physical stability.

In the background art the insulin solutions to be spray-dried are reported to have a pH near the neutrality obtained by using citrate buffer (pH 6.7 ± 0.3, WO-A-95/24183) or above neutral (WO 00/00176). Insulin for pulmonary therapy in patent WO-A-95/24183 was declared as prepared by spray drying solutions in physiologically acceptable buffer such as citrate buffer at pH between 2.0-9.0, but the powder described in the example was made by spray drying a solution at pH 6.7 ±0.3. In patent WO 00/00176 the spray drying of a true solution over the isoelectric point of insulin was described.

The present invention is based on the surprising discovery that the spray drying of dear, concentrated, aqueous solutions of insulin having pH lower than the isoelectric point of the hormone (5.4), produces very high respirable dry powders. These powders can be obtained from un-buffered solutions and were never prepared before. The spray drying of insulin solution under the isoelectric point and therefore, in acidic conditions and without the use of permanent buffering agents, was not previously considered likely for stability and respirability reasons. On the contrary, insulin microparticles produced by spray drying acidic solutions of the hormone resulted in powders particularity suitable for lung administration, because they exhibit a high respirable dose. In addition, the stability was acceptable in refrigerated conditions but, when the powder was manufactured from a solution in acetic acid, the stability resulted surprisingly very high also in normal conditions. Since no permanent buffers were used, it is also expected that these powders inhaled at the therapeutic doses do no modify the alveolar surfactant pH.

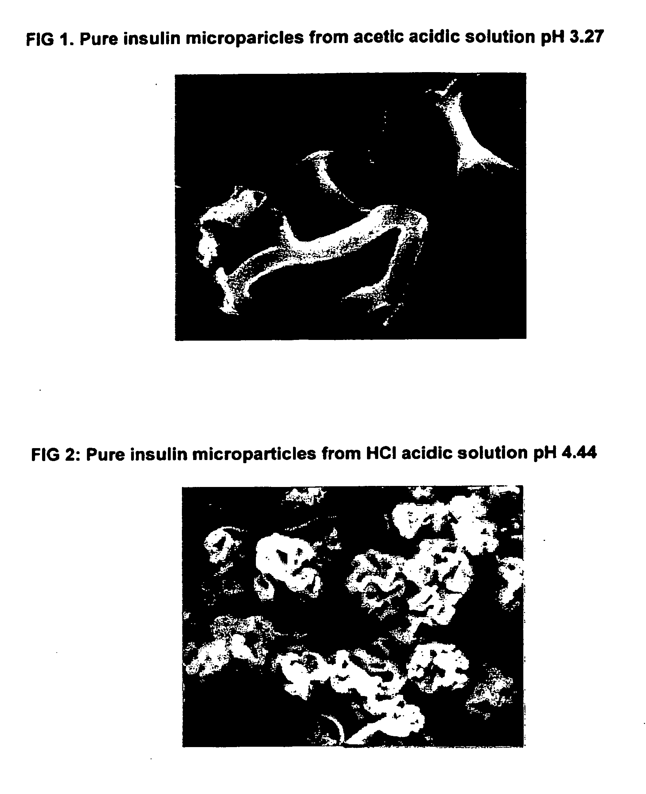

As described in previous patent (WO 00/00176) concerning insulin formulations obtained by spray drying, the neutralisation procedure of Insulin solutions to pH above 7.0 results in the production of microparticles with "dimpled surface that may be beneficial" in term of respirability. We reproduced these particles made at pH 7.45 in order to focus the "dimple" shape. The procedure applied is the one described in the WO 00/00176 patent. The microparticles obtained according to the present patent are much more than dimpled since they are corrugated or raisin like (Figures 1 and 2). In addition, they are non-cohesive with favourable aerodynamic size and density characteristics.

Similar shapes were observed independently of the acids employed for the preparation of insulin solutions. However, we unexpectedly discovered that when the volatile organic acetic acid is used to dissolve insulin, powders obtained by spray drying from these low pH solutions have lost acidity. In fact, the dissolution of these powders in distilled degassed water gives rise to a solution having pH higher than the value of the original solution. This fact made this powder chemically very stable during storage.

Therefore, the novelty of the powders described in this patent is based on the finding that the peculiar corrugated microparticles obtained by spray-drying insulin acidic solution are micronized, free flowing and with low tapped density. The microparticles are essentially amorphous and characterized by a shape defined corrugated or raisin like. This particle shape makes the powders not cohesive since the microparticles maintain their individuality and do not agglomerate. In addition, they show substantially no losses of activity if stored in refrigerated conditions, but when they are prepared from acetic acid, the insulin powders are very stable also at room conditions (25°C). In particular, we surprisingly discover that the acetic acid solution of insulin at pH 3.3 after spray drying gave rise to a powder that re-dissolved in distilled degassed water at 1 mg/ml showed a pH of 6.4. Surprising this powder shows a superior stability at 25°C allowing the preparation to be used and dispensed at room conditions, in comparison with Insulin spray dried powders prepared with HCl that must be stored at refrigerated conditions.

Differently from the common technique, such spray dried powders characterized a peculiar insulin particle shape, are produced from clear, volatile un-buffered solutions having acidic pH values between 3.0 - 4.5, lower than the isolelectric point of insulin. The use of acidic solutions avoids the risk of precipitation induced by increasing the pH above insulin isoelectric point up to neutrality, but more interestingly provides a structure to dried product that surprisingly is very useful for the aerosolization. In fact, these powders other than to be micronized, are not cohesive, quite free flowing and easy meterable in the DPI. These physical properties, together with the favourable aerodynamic behaviour due to the size, shape and density of the particles, determine an unexpected and surprisingly high respirability.

Finally, the powders contain residual moisture enough to prevent excessive degradation and they can be stored at normal humidity and temperature conditions when prepared from volatile acetic acid.

### FORMULATION ASSAY

The activity of all formulations has been estimated by HPLC. According to the official monographs for "Insulin preparations" (USP 26 and European Pharmacopoeia 4th Edition, page. 1368-1381) HPLC performed in different conditions gives information about potency (according USP 26, potency is evaluated in comparison to a certified standard, test described under "ASSAY"), purity (quantification of the "related proteins") and about the presence of covalent aggregation (called "impurities with molecular masses greater than that of insulin"), both in European Pharmacopoeia 4th Edition.

Pharmacopoeias limits and specifications for the insulin preparations are: A21 desamido not more than 5% of total area of peaks, other not more than 6%. Impurities with molecular masses greater than that of insulin: not more than 2% of total area of peaks.

Aerodynamic diameter was assessed using the Andersen Cascade Impactor. The percentage of mass less than the stated aerodynamic diameter versus aerodynamic diameter is then plotted on a log probability paper (USP 26 page 2123). Respirability of the described formulations is derived from the data plotted as described above considering the mass less than 5 microns as respirable. Packing properties have been studied using tap density measurements. According to the official monograph, tap densities has been evaluated after 1250 taps (USP 26) employing a 10 ± 0.05 ml cylinder filled with the powder.

### DESCRIPTION OF THE INVENTION

The microparticles of this invention contain drug substantially free of excipients, but in certain conditions mixture of drug and diluent such as mannitol can be used. Substantially free of excipients means that the microparticles of the invention can include process-linked component as hydrochloric acid or acetic acid and eventually their sodium salts in case of pH adjustement up to about 10% of the total solids. The main advantage of the use of substantially excipient-free formulations is that each dose can contain a large amount of the active. Buffer salts like citrate are not necessary both for solubility and stability of the microparticles described under the invention. In all previous patents insulin final solutions for spray drying are reported to have a pH near the neutrality using citrate buffer (6.7 ± 0.3, WO-A-95/24183) or above neutral (WO 00/00176). The procedure at pH 6.7 did not allow the microparticles to be produced from a dear solution of insulin and from the dear solution above the neutrality the particles had a surface smooth or "dimpled", morphological characteristics, considered as beneficial for inhalation. Nevertheless, the use of mild acidic solutions leads to reproducible production of corrugated or raisin like microparticles, without affecting the insulin stability if property stored.

The solutions of insulin are spray dried in a conventional spray drying apparatus; even rotary atomization, pressure atomization and two-fluid atomization can be employed as spraying process. No particular restrictions are placed on the gas used to dry the sprayed materials. Filtered air is used in the manufacturing methods described below. The temperature of the inlet of the gas used to dry the sprayed materials should be chosen so that it does not cause degradation of the active. The range may vary between 50°C and 200°C. The temperature of the outlet gas used may vary between 30° and 100°C, preferably in the range 40°C and 60°C. This has been found to affect more than the inlet temperature set up the degree of degradation of the dried product. The fact that inlet and outlet temperature above 50°C can be used has been noted and reported (US 6582728). Insulin solutions were prepared by dissolving from 5 to 20 mg of the hormone per milliliter in 10⁻² M hydrochloric acid solutions or 0.4M diluted acetic acid solutions (pH 2.6), diluting with distilled water and adding if necessary an adequate amount of NaOH solution to pH between 3.0 and 4.5, in any case avoiding insulin precipitation. Excipients such as polyalcohols can be added before pH correction. A Mini Spray Drier Büchi, model 191 (Buchi, Labortechnik AG, Flawil, Switzerland) equipment was used. The inlet air (drying gas) had initially, before heating, a relative humidity of about 30-70%. The nozzle was provided with an orifice of 0.7 or 1.0 mm internal diameter. The atomizing gas was filtered compressed air. The spray drier was equipped with a standard cyclone. The used range of the spray drying parameters was: feed flow rate 180-360 ml/h; nozzle gas flow rate 500-800 l/h; inlet air temperature < 140 °C, producing an outlet temperature of 40-60 °C; aspirator capacity ≤ 35 m³/h (100 % setting). The selected parameter range allowed to obtain an original and new powder with good flow and packing properties, having particle size, shape and density in the respiratory range and with a respirable fraction higher than 80%, with a moisture content in the range of 2 to 8% and a pH of reconstituted solution near neutrality when the acidic solution to spray dry was made with acetic acid, thus preventing insulin degradation.

### EXAMPLES

According to the object of the invention, the following dispersible dry powder formulations were prepared as described. All formulations meet the strict specifications for content and purity required for pharmaceutical products.

### Example 1 (not according to the invention)

### A) Formulation.

2550 mg of highly purified bovine insulin was dissolved in 200 ml of aqueous 10⁻² M hydrochloric acid. The solution was added with 100 ml of distilled water and then with 450 mg of mannitol under stirring to give a final solid concentration of 10 mg per ml (8.5 mg bovine insulin per ml). The pH of the dear solution was adjusted to 4.35 using NaOH 0.1 N dropwise.

### B) Spray Drying.

This solution was filtered and was spray dried using a Mini spray drier Büchi, model 191 (Büchi, Labortechnik AG, Flawil, Switzerland) under the following process conditions: feed flow rate 195 ml/h; nozzle gas flow rate 600 nl/h; atomizing nozzle diameter 1.0 mm; inlet air temperature 120 °C. producing an outlet temperature of 42 °C; aspirator capacity 100% setting. The yield was about 60%.

### C) Characterization.

The collected powders were assayed by HPLC for covalent aggregation and degradation products (A21 desamido insulin) according to the European Pharmacopeia 4 (pag. 1368-1381), by Scanning Electron Microscopy (SEM) for morphology investigations, by laser diffraction for particle size distribution and Andersen Cascade Impactor for respirable fraction evaluation.

The potency was 22.6 Ul/mg, the related proteins were 0.5% and the impurities with molecular mass greater than insulin were 0.55%. The powder contained approximately 5.8% moisture. The particle size distribution of the powder was determined to be 2.33 (d₁₀), 3.62 (d₅₀) and 5.68 (d₉₀) microns as volume diameter. The respirability (mass less than 5 microns) derived from Andersen Cascade Impactor data was high as 85.7%. The packing properties measured as tapped density (European. Pharmacopoeia 4th Edition) was 0.2 g/cm³. The powder dissolved in distilled and degassed water gave rise to a pH of 4.4. Weighed amounts of powder were then placed into separated glass vials and stored at - 18°C, in a refrigerator at 5 ± 3°C and at room temperature and humidity (25 ± 3°C and 65 ± 5% RH respectively) and analysed at different times by HPLC for stability.

### Example 2

### A): Formulation.

975 mg of bovine insulin was dissolved in 95 ml of aqueous diluted acetic acid (pH 2.6). The solution was added with 0.6 ml of NaOH 1M. The pH of the clear solution was 3.27.

### B): Spray drying Process.

This solution was filtered and subsequently spray dried using a Mini spray drier Büchi, model 191 (Büchi, Labortechnik AG, Flawil, Switzerland) under the following process conditions: feed flow rate 200 ml/h; nozzle gas flow rate 500 nl/h; atomizing nozzle diameter 1.0 mm; inlet air temperature 130 °C, producing an outlet temperature of 55 °C; aspirator capacity 100% setting. The yield was about 50%.

### C) Characterization.

The collected powders were assayed by HPLC for covalent aggregation and degradation products (A21 desamido insulin) again according to the European Pharmacopeia and by the already quoted assays. The potency was 28.9 Ul/mg, the related proteins were 0.6% and the impurities with molecular mass greater than insulin was 0.33%. The particle size distribution was determined to be 4.06 (d₁₀), 4.36 (d₅₀) and 4.93 (d₉₀) microns as volume diameter. The respirability (mass less than 5 microns) derived from Andersen Cascade Impactor data was high as 83.9%. The tapped density, index of packing properties, (Eu.Pham), was 0.1 g/cm³. The powder dissolved in distilled and de-gased water gave rise to a pH of 6.4. Microparticles exhibit a raisin-like shape (Figure 1).

### Example 3 (not according to the invention)

### A) Formulation.

1750 mg of highly purified bovine insulin was dissolved in 120 ml of aqueous 10⁻² M hydrochloric acid. The solution was added dropwise with.0.7 ml of 1 N NaOH to give a final pH of about 4.44. The solution is clear and contains about 15 mg per ml of solids.

### B) Spray Drying Process.

This solution was filtered and then spray dried using a Mini spray drier Büchi under the following process conditions: feed flow rate 195 ml/h; nozzle gas flow rate 600 nl/h; atomisng nozzle diameter 1.0 mm; inlet air temperature 120 °C, producing an outlet temperature of 46 °C; aspirator capacity 100% setting. The yield was about 55%. C) Characterization.

The microparticles were assayed by HPLC for covalent aggregation and degradation products (A21 desamido insulin) according to the European Pharmacopoeia, by SEM for morphology investigations, by laser diffraction for particle size distribution and by Andersen Cascade Impactor for respirable fraction evaluation.

The potency was 27.1 Ul/ mg, the related proteins were 0.7% and the impurities with molecular mass greater than insulin were 0.4%. The formulation contained approximately 4.7% moisture

The particle size distribution was determined to be 3.12 (d₁₀), 4.72 (d₅₀) and 7.24 (d₉₀) microns as volume diameter. The powder dissolved in distilled and degassed water gave rise to a pH of 4.7.

The respirability (mass less than 5 microns) derived from Andersen Cascade Impactor data was high as 90.4%.

Weighed amounts of powder were then placed into separated vials and stored at - 18°C, in a refrigerator at 5 ± 3°C and at room temperature and humidity (25 ± 3°C and 65 ± 5% RH respectively) and analyzed at different times by HPLC for stability.

## Claims

1. Microparticles stable at room temperature of insulin, optionally in association with excipients selected from the group consisting of saccharides, polysaccharides, aminoacids, phospholipids and polyalcohol,
said microparticles:
• being obtained by spray drying a clear aqueous solution of insulin having an acid pH under the isoelectric point (5.4) of insulin and a concentration of insulin in amounts of from 5 to 100 mg/ml,
• showing a d90 volume diameter lower than 9 µm,
• more than 80% of them exhibiting an aerodynamic diameter lower than 5 µm,
• containing less than 10% by weight of salts,
**characterised in that** said aqueous solution of insulin to be spray dried is prepared in an unbuffered aqueous solution of acetic acid.

2. Microparticles according to claim 1 having a tapped density lower than 0.2 g/cm³.

3. Microparticles according to claim 2, wherein said excipient is mannitol.

4. Microparticles according to anyone of claim 1-3 containing insulin in amorphous form.

5. Pharmaceutical compositions suitable to be inhaled containing the microparticles according to anyone of claims 1-4

6. The pharmaceutical compositions according to claim 5 consisting of the microparticles according to anyone of claims 1-4.

## Patentansprüche

1. Mikropartikel von Insulin, die bei Zimmertemperatur stabil sind, optional in Verbindung mit Exzipienten, die aus der Gruppe ausgewählt sind, die aus Sacchariden, Polysacchariden, Aminosäuren, Phospholipiden und Polyalkohol besteht,
wobei die genannten Mikropartikel:
• durch Sprühtrocknen einer klaren wässrigen Lösung von Insulin mit einem sauren pH-Wert unter dem isoelektrischen Punkt (5,4) von Insulin und mit einer Konzentration von Insulin in Mengen von 5 bis 100 mg/ml erhalten werden,
• einen d90-Volumen-Durchmesser niedriger als 9 µm zeigen,
• zu mehr als 80 % einen aerodynamischen Durchmesser niedriger als 5 µm zeigen,
• weniger als 10 Gew.-% Salze enthalten,
**dadurch gekennzeichnet, dass** diegenannte wässrige Lösung von Insulin, die sprühgetrocknet werden soll, in einer ungepufferten wässrigen Lösung von Essigsäure zubereitet wird.

2. Mikropartikel gemäß Anspruch 1 mit einer Rütteldichte niedriger als 0,2 g/cm³.

3. Mikropartikel gemäß Anspruch 2, wobei dergenannte Exzipient Mannitol ist.

4. Mikropartikel gemäß einem der Ansprüche 1-3, die Insulin in amorpher Form enthalten.

5. Arzneimittelzusammensetzungen, die dafür geeignet sind, inhaliert zu werden, die die Mikropartikel gemäß einem der Ansprüche 1-4 enthalten.

6. Arzneimittelzusammensetzungen gemäß Anspruch 5, die aus den Mikropartikeln gemäß einem der Ansprüche 1-4 bestehen.

## Revendications

1. Microparticules stables à température ambiante de l'insuline, optionnellement en association avec des excipients sélectionnés dans le groupe consistant en saccharides, polysaccharides, acides aminés, phospholipides et polyalcools, les microparticules:
• étant obtenues par séchage par atomisation d'une solution aqueuse incolore d'insuline ayant un pH acide sous le point isoélectrique (5,4) d'insuline et une concentration d'insuline en des quantités de 5 à 100 mg/ml,
• représentant un diamètre de volume d90 inférieur à 9µm,
• plus que 80% de celles-ci ayant un diamètre aérodynamique inférieur à 5 µm,
• contenant moins que 10% en poids de sels,
**caractérisées en ce que** ladite solution aqueuse d'insuline à sécher par atomisation est préparée dans une solution aqueuse non tamponnée d'acide acétique.

2. Microparticules selon la revendication 1 ayant une densité après tassement inférieure à 0,2 g/cm³.

3. Microparticules selon la revendication 2, où ledit excipient est le mannitol.

4. Microparticules selon l'une quelconque des revendications 1 à 3, contenant de l'insuline sous forme amorphe.

5. Compositions pharmaceutiques aptes à être inhalées contenant les microparticules selon l'une quelconque des revendications 1 à 4.

6. Compositions pharmaceutiques selon la revendication 5, consistant en microparticules en accord avec l'une quelconque des revendications 1 à 4.
